# EUROPEAN PATENT APPLICATION

(11) **EP 1 698 370 A1**
(43) Date of publication of application: **06.09.2006**
(21) Application number: 06003798.3
(22) Date of filing: 24.02.2006
(51) Int. Cl.: A61M 25/09, A61M 25/01

(54) **Guide wire**

(30) Priority: 02.03.2005 JP 2005057912; 18.01.2006 JP 2006010188
(71) Applicant: TERUMO KABUSHIKI KAISHA, Tokyo (JP)
(72) Inventor: Satou, Hideo, Fujinomiya-Shi, Shizuoka (JP); Aimi, Youki, Fujinomiya-Shi, Shizuoka (JP); Itou, Yutaka, Fujinomiya-Shi, Shizuoka (JP)
(74) Representative: Casalonga, Axel

(57) **Abstract**

A guide wire (1) includes a core member (2) having at least one projection (4) projecting relative to the axis. The guide wire includes a coil (3) having a distal end portion and a proximal end portion covering at least a distal end portion of the core member. The coil is fixed to the projection(s).

## Description

The present invention relates to a guide wire, particularly a guide wire for use in introducing a catheter into a body lumen such as a blood vessel.

Guide wires are used to guide a catheter in treatment of cites at which open surgeries are difficult or which require low invasiveness to the body, for example, PTCA (Percutaneous Transluminal Coronary Angioplasty), or in examination such as cardioangiography. A guide wire used in the PTCA procedure is inserted, with the distal end projecting from the distal end of a balloon catheter, into the vicinity of a target angiostenosis portion together with the balloon catheter, and is operated to guide the distal end portion of the balloon catheter to the target angiostenosis portion.

A guide wire used to insert a catheter into a blood vessel complicatedly curved requires appropriate flexibility and restoring performance against bending, pushability and torque transmission performance (generically called "steerability") for transmitting an operational force from the proximal end portion to the distal side, and kink resistance (often called "resistance against sharp bending").

To obtain appropriate flexibility as one of the above-described performances, there has been known a guide wire configured such that a metal coil having flexibility is provided around a small-sized core member at the distal end of the guide wire, or a guide wire including a plastic coating on a core member made from a superelastic material such as an Ni-Ti alloy for improving the flexibility and restoring performance. The guide wire having the coil has the difficulty that, in fixing the coil to the core member of the guide wire, it is not always easy to fix the core member at the center of the coil.

Japanese Patent Laid-Open No. Sho 60-168466 discloses a guide wire in which two coils formed of different materials are disposed in series and are fixed to a core member by screw engagement with each other. Japanese Patent Laid-Open No. Hei 10-43307 discloses a guide wire such that two coils are joined to each other on a centering mold and thereafter the mold is removed. In addition, Japanese Patent Laid-Open No. Hei 6-327775 discloses a guide wire in which two coils are connected to each other through a connection coil. Furthermore, US Patent No. 5,666,969 discloses a guide wire in which two coils are coupled to each other through a spacer.

However, none of the above-mentioned patent references can be said have succeeded in solving the above-mentioned problem.

Accordingly, it is an object of the present invention to provide a guide wire in which a coil can be disposed substantially concentrical with a core member, of which a distal end portion shows an even and favorable deformation at the time of insertion into a living body, and which is excellent in operationality.

In order to attain the above object, according to the present invention, there are provided the followings.

A guide wire provides a core member having at least one projection projecting concentrically relative to an axis, and a coil having a distal end portion, a proximal end portion, and an intermediate portion and covering at least a distal side portion of the core member, wherein the coil is fixed to the projection. The projection preferably has a cylindrical portion. The projection preferably has a concave portion at least on one of the distal side and the proximal side thereof. The projection preferably has tapered portions on the distal side and the proximal side thereof. A projecting portion of the projection is preferably trapezoidal in sectional shape. A projecting portion of the projection is preferably mount-like in sectional shape. The projection has an outside diameter substantially equal to the inside diameter of the coil. The projection is preferably provided at a portion between the distal end portion and the proximal end portion of the coil and/or at the distal end portion of the coil. The coil preferably includes a distal side coil and a proximal side coil, and the projection fixes a proximal end portion of the distal side coil and a distal end portion of the proximal side coil to the core member. The distal side coil and the proximal side coil are preferably different in physical properties. A proximal end portion of the distal side coil and a distal end portion of the proximal side coil are preferably entangled with each other. The distal side coil and the proximal side coil have preferably substantially equal outside diameters and difference inside diameters, and the projection has a small-diameter portion and a large-diameter portion corresponding to the difference in inside diameter between the coils. The projection preferably has a concave portion on at least one of the distal side and the proximal side thereof. Two or more projections are preferably provided. The projection preferably has a distal side projection and a proximal side projection, a proximal end portion of the distal side coil and a distal end portion of the proximal side coil are joined to each other, and the joint portion of the distal side coil and the proximal side coil is located between the distal side projection and the proximal side projection. The coil is preferably fixed to the projection by an adhesion material. The coil is preferably fixed to the projection by welding. The distal end of the coil is preferably fixed to the distal end of the core member, the proximal end of the coil is fixed to a portion spaced by a predetermined distance to the proximal side from the distal end of the core member, and the core member extends from the proximal end portion of the coil to constitute a main body portion of the guide wire. A shaping member preferably is secured to the projection.

The guide wires according to the present invention each have a portion for fixing the coil substantially concentrically to the core member, at least at a portion of the coil. Therefore, the gap between the core member and the coil is reduced, so that the coil can be fixed to the core member substantially concentrically, and a distal end portion of the guide wire shows an even and favorable deformation at the time of insertion into a living body.

The above and other objects, features and advantages of the present invention will become more apparent from the following description and appended claims, taken in conjunction with the accompanying drawings, in which:
Fig. 1 is a partly omitted schematic front view of an embodiment of a guide wire according to the present invention;
Fig. 2 is a partial enlarged sectional view of another embodiment of the guide wire according to the present invention;
Fig. 3 is a partial enlarged sectional view of another embodiment of the guide wire according to the present invention;
Fig. 4 is a partial enlarged sectional view of another embodiment of the guide wire according to the present invention;
Fig. 5 is a partial enlarged sectional view of another embodiment of the guide wire according to the present invention;
Fig. 6 is a partial enlarged sectional view of another embodiment of the guide wire according to the present invention;
Fig. 7 is a partial enlarged sectional view of another embodiment of the guide wire according to the present invention;
Fig. 8 is a partial enlarged sectional view of another embodiment of the guide wire according to the present invention;
Fig. 9 is a partial enlarged sectional view of another embodiment of the guide wire according to the present invention;
Fig. 10 is a partial enlarged sectional view of another embodiment of the guide wire according to the present invention;
Fig. 11 is a partial enlarged sectional view of another embodiment of the guide wire according to the present invention;
Fig. 12 is a partial enlarged sectional view of another embodiment of the guide wire according to the present invention;
Fig. 13 is a partial enlarged sectional view of another embodiment of the guide wire according to the present invention;
Fig. 14 is a partial enlarged sectional view of another embodiment of the guide wire according to the present invention;
Fig. 15 is a partial enlarged sectional view of another embodiment of the guide wire according to the present invention;
Fig. 16 is a partial enlarged sectional view of another embodiment of the guide wire according to the present invention;
Fig. 17 is a sectional view along line A-A of Fig. 16;
Fig. 18 is a sectional view showing a modified example of Fig. 17;
Fig. 19 is a partial enlarged sectional view of another embodiment of the guide wire according to the present invention; and
Fig. 20 is a sectional view along line B-B of Fig. 19.

The guide wire according to the present invention will be described using embodiments shown in the drawings.

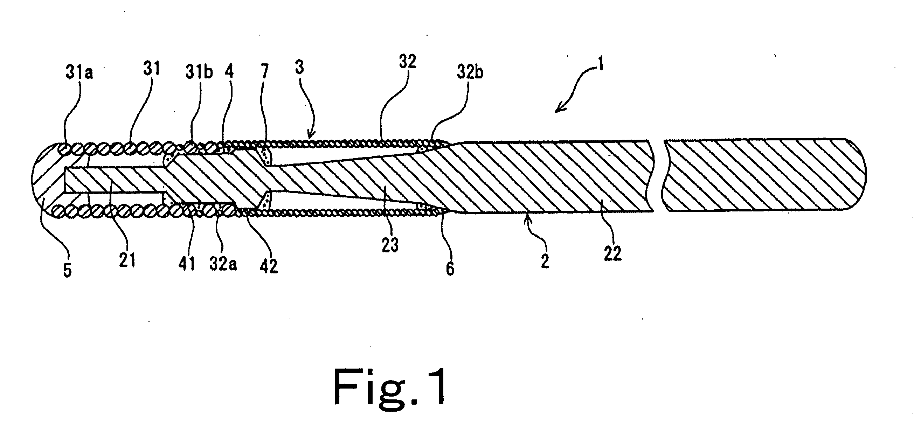

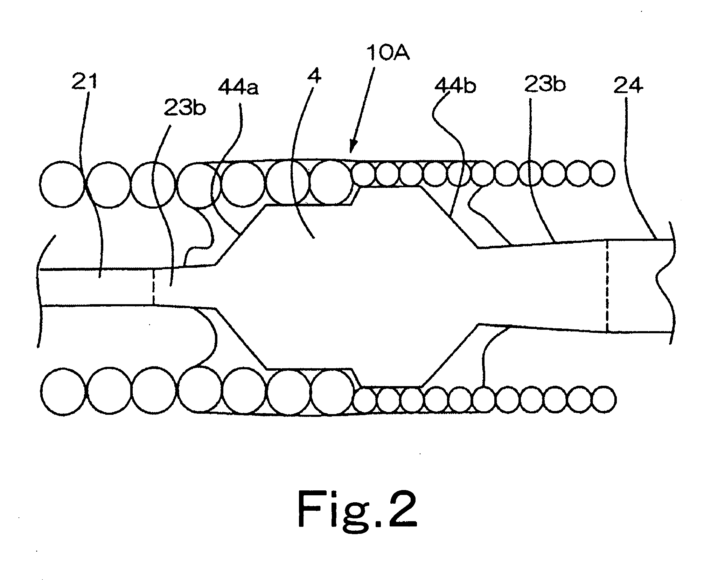

Fig. 1 is a partly omitted sectional view of an embodiment of the guide wire according to the present invention. Figs. 2 to 11 are partial enlarged sectional views of other embodiments of the guide wire according to the present invention.

The guide wire 1 according to the embodiment shown in Fig. 1 includes a core member 2 having at least one projection 4 projecting substantially concentrically relative to the axis, and a coil 3 having a distal end portion and a proximal end portion and covering at least a distal side portion of the core member 2. The coil 3 of the guide wire 1 is fixed to the projection 4. The projection 4 is for fixing a proximal end portion 31b of a distal side coil 31 and a distal end portion 32a of a proximal side coil 32 to the core member 2. The distal end of the coil 3, or the distal end 31a of the distal side coil 31 in this embodiment, is fixed to a distal end portion of the core member 2. The proximal end portion of the coil 3, or the proximal end 32b of the proximal side coil 32 in this embodiment, is fixed to a portion spaced by a predetermined distance to the proximal side from the distal end of the core member 2. Namely, in the guide wire 1 according to this embodiment, a distal end portion of the coil 3 is fixed to the distal end of the core member 2, a proximal end portion of the coil 3 is fixed to the portion spaced by the predetermined distance to the proximal side from the distal end of the core member 2, and the core member 2 extends from the proximal end portion of the coil 3 to constitute a main body portion of the guide wire 1.

The whole length of the guide wire 1 is preferably about 300 to 4500 mm, more preferably about 1000 to 3000 mm.

The core member 2 includes a main body portion 22, a tapered portion 23 and a distal end portion 21 in this order from the proximal side. The main body portion 22 is an elongate portion having a substantially constant outside diameter and extending in the distal direction, and the distal end portion 21 is smaller than the main body portion 22 in diameter. In this embodiment, the distal end portion 21 extends toward the distal side while having roughly the same diameter. The tapered portion 23 is formed between the distal end portion 21 and the main body portion 22.

The outside diameter of the guide wire 1 at the main body portion 22 is preferably 0.2 to 1.8 mm, more preferably about 0.3 to 1.6 mm. The outside diameter of the distal end portion 21 is preferably 0.05 to 1.6 mm, and the length thereof is 10 to 500 mm, preferably 20 to 300 mm. The distal end portion 21 may be more flexible on the distal side; for example, the distal portion 21 has been heat treated so that its flexibility is gradually increased as the distal end is approached.

Examples of the material constituting the core member 2 include various metallic materials such as superelastic alloys such as an Ni-Ti based alloy, stainless steel, cobalt-based alloys, piano wire, etc. A configuration may be adopted in which a comparatively flexible wire of an Ni-Ti alloy or the like is used as a distal side core material, while a wire higher in rigidity than the distal side core member, for example, a stainless steel is used as a proximal side core member, and both the core members are joined to each other to constitute the core member 2. The distal side core member and the proximal side core member may be joined to each other permanently by connecting tube, soldering, brazing, or welding.

The whole length of the coil 3 is 10 to 500 mm, preferably 20 to 300 mm, and the outside diameter of the coil 3 is 0.2 to 1.8 mm, preferably 0.3 to 1.6 mm. In addition, the coil 3 covers the distal end portion 21 and the tapered portion 23 of the core member 2. The distal end of the coil 3 is fixed to the distal end of the core member 2, and the proximal end of the coil 3 is attached to a proximal end portion of the tapered portion 23 of the core member 2 by a brazing filler metal 6 or the like.

In this embodiment, the coil 3 is composed of the distal side coil 31 and the proximal side coil 32, as shown in Fig. 1. Further, the proximal end portion 31b of the distal side coil 31 and the distal end portion 32a of the proximal side coil 32 are entangled with each other. With both of them thus entangled with each other, both of them are prevented from being separated from each other. The distance over which both of them are entangled with each other is preferably about 0.1 to 2 mm.

In addition, the distal side coil 31 and the proximal side coil 32 are preferably different in physical properties. The physical properties of both coils may be selected variously according to the purpose of the guide wire. Examples of configurations which can be adopted include a configuration in which the distal side coil is more flexible than the proximal side coil, and a configuration in which the distal side coil is higher in radioopacity than the proximal side coil. The flexibility of the coil can be varied, for example, by varying the winding mode of the coil, namely, either providing a fixed gap between loops of the elementary wire of the coil or winding the elementary wire so that the loops are in close contact with each other, by varying the diameter of the elementary wire of the coil, or by changing the elementary wire material of the coil. In this embodiment, the elementary wire diameter of the distal side coil 31 is greater than that of the proximal side coil 32, and the distal side coil 31 is higher in radioopacity than the proximal side coil 32. The distal side coil 31 and the proximal side coil 32 are substantially equal in outside diameter. The inside diameter of the distal side coil 31 is smaller than that of the proximal side coil 32.

The length of the distal side coil 31 is preferably about 3 to 60 mm, and the length of the proximal side coil 32 is preferably about 10 to 40 mm.

Examples of materials which can be used for forming the coils include superelastic alloys such as Ni-Ti based alloys, stainless steel, cobalt based alloys, and noble metals such as gold and platinum. The distal side coil and the proximal side coil may be formed from the same material or from different materials. Where different materials are used for the coils, for example, a configuration may be adopted in which a noble metal such as gold and platinum is used for the distal side coil whereas stainless steel is used for the proximal side coil, or another configuration may be adopted in which a superelastic alloy is used for the distal side core whereas stainless steel is used for the proximal side core.

The distal end of the coil 3 is fixed to the distal end of the core member 2 by a brazing filler metal 5. The distal end of the guide wire 1 which is formed of the brazing filler metal 5 is a hemispherically shaped distal end portion. The hemispherically shaped distal end means that the distal end is substantially formed to have a curved surface, the shape including a hanging bell-like shape, a bullet-like shape, etc.

The core member 2 is provided at its distal end portion 21 with the projection 4 projecting substantially concentrically relative to the axis. With the projection 4 thus provided, the coil 3 can be fixed to the core member 2 substantially concentrically, at least at a portion in the longitudinal direction of the coil 3.

The length of the projection 4 is preferably about 0.1 to 2.0 mm. The whole length of the projection 4 is preferably greater than the outside diameter of the core member 2 which is larger in diameter and which is located either on the distal side or on the proximal side of the projection 4, since in this configuration any bending stress exerted on the relevant portion would not be concentrated on the portion.

While the projection 4 is located on the distal side relative to the intermediate portion in the longitudinal direction of the coil 3 in this embodiment, the projection 4 may be provided at a substantially intermediate portion in the longitudinal direction of the coil 3 or on the proximal side relative to the intermediate portion in the longitudinal direction of the coil 3. The projection 4 is located at a position spaced by a predetermined distance to the distal side or the proximal side of the coil 3. In addition, a plurality of such projections 4 may be provided, as will be described below. For example, a configuration may be adopted in which the projections 4 are disposed at positions spaced by a predetermined distance to the distal side and the proximal side from the portion where the distal side coil 31 and the proximal side coil 32 are joined to each other, and the distal side coil 31 and the distal side projection are fixed to each other, and the proximal side coil 32 and the proximal side projection are fixed to each other, by use of an adhesion material such as a brazing filler metal.

In the guide wire 1 in this embodiment, the projection has a cylindrical portion, as shown in Fig. 1. The projection 4 has tapered portions on the distal side and the proximal side thereof. In Fig. 1, the distal side tapered portion and the proximal side tapered portion are inclined at substantially the same angle against the axis of the core member and in the opposite senses. The angle of the distal side tapered angle may be less than the angle of the proximal side tapered portion. Or, the angle of the proximal side tapered portion may be less than the angle of the distal side tapered portion. The projection 4 includes a small diameter portion 41 corresponding to a proximal end portion of the distal side coil 31, or to the entangled portion of the distal side coil and the proximal side coil in the embodiment shown in the figure, and a large diameter portion 42 corresponding to the proximal side coil 32. In the guide wire 1, the projection 4 is provided at a position corresponding to the entangled portion of the distal side coil 31 and the proximal side coil 32. The outside diameter of the small diameter portion 41 of the projection 4 is substantially equal to the inside diameter of the distal side coil 31. The outside diameter of the large diameter portion 42 of the projection 4 is substantially equal to the inside diameter of the proximal side coil 32. The difference in outside diameter between the large diameter portion 42 and the small diameter portion 41 is substantially equal to the difference in inside diameter between the distal side coil 31 and the proximal side coil 32. It is preferable that the outside diameter of the large diameter portion 42 of the projection is greater than the inside diameter of the distal side coil 31. This ensures that, as shown in Fig. 1, the proximal end of the distal side coil 31 abuts on an end portion of the large diameter portion 42 of the projection, so that the distal side coil 31 can be prevented from moving toward the proximal side. Furthermore, the work for fixing the coil to the core member is made easier. A gradually reduced diameter portion is located between the large diameter portion 42 and the small diameter portion 41. The gradually reduced diameter portion is preferably a tapered portion. The gradually reduced diameter portion may be a concave portion. The diameter of the projection 4 is reduced in a tapered manner from the large diameter portion 42 to the small diameter portion 41. The length of the large diameter portion 42 is preferably smaller than the outside diameter of the large diameter portion 42. The length of the small diameter portion 41 is preferably smaller than the outside diameter of the small diameter portion 41.

The projection 4 and the core member 2 are preferably formed from the same material and a single unitary part, as in this embodiment. The projection 4 and the core member 2 may be formed from the same material and different parts. The projection 4 and the core member 2 may be formed from the different material and different parts. The projection 4 in this embodiment is configured as a part of the core member 2. The projection 4 can be formed at the time of forming the tapered portion 23 and the distal end portion 21 of the core member 2. The method of forming is preferably mechanical grinding, particularly centerless grinding.

The coil 3 is fixed to the projection 4 by a brazing filler metal 7, which is an adhesion material. Examples of the adhesion material include not only the brazing filler metals (inclusive of solders) but also resin-made adhesives. The coil 3 is fixed to the projection 4 through the adhesion material. The adhesion material is made to fill the gap between the projection 4 and the core member 2. Depending on the material of the core member 2, a metal layer may be provided on the surface of the projection 4, for enhancing the adhesion to or affinity for the brazing filler metal. The material constituting the metal layer can be so selected that the adhesion between the metal layer and the brazing filler metal 7 is higher than the adhesion between the core member 2 and the brazing filler metal 7. The metal layer may be formed by plating.

The coil 3 may be fixed to the projection 4 by welding. Laser welding and TIG welding can be used as the welding. The outside surface of at least one of the large diameter portion 42 and the small diameter portion 41 may be covered with a metallic annular member.

With the projection 4 thus provided, the gap between the core member and the coil is reduced, so that the coil 3 can be fixed to the core member substantially concentrically, and, at the time of insertion into a living body, the distal end portion of the guide wire shows an even and favorable deformation, and smooth torque transmission performance can be realized. In addition, the brazing filler metal is prevented from flowing excessively in the axial direction of the guide wire at the time of fixing, so that the length of the fixed portion of the coil and the core member can be reduced.

Fig. 2 is a partial enlarged sectional view of another embodiment of the guide wire according to the present invention. Fig. 2 is an enlarged sectional view of the projection 4 and the vicinity thereof. This guide wire 10A is the same as the above-described guide wire in basic configuration; therefore, the differences therebetween will be described principally, and, for the other points, the above description can be referred to.

In the embodiment shown in Fig. 2, the projection 4 is provided at the tapered portion of the core member 2. The core member 2 has a main body portion (not shown), a proximal side tapered portion (not shown), a constant outside diameter portion 24, a distal side tapered portion 23b, and a distal end portion 21. The projection 4 has tapered portions on the distal side and the proximal side thereof. In Fig. 2, the distal side tapered portion 44a and the proximal side tapered portion 44b are inclined at substantially the same angle against the axis of the core member and in the opposite senses. The angle of the distal side tapered portion 44 may be less than the angle of the proximal side tapered portion 44. The angle of the distal side tapered portion 44a of the projection 4 is greater than the angle of the distal side tapered portion 23b of the core member 2. The outside diameter may be gradually decreased from the distal side tapered portion 44a to the distal side tapered portion 23b. A proximal end portion of the distal side coil 31 and a distal end portion of the proximal side coil 32 are fixed in the state of being adjacent to each other.

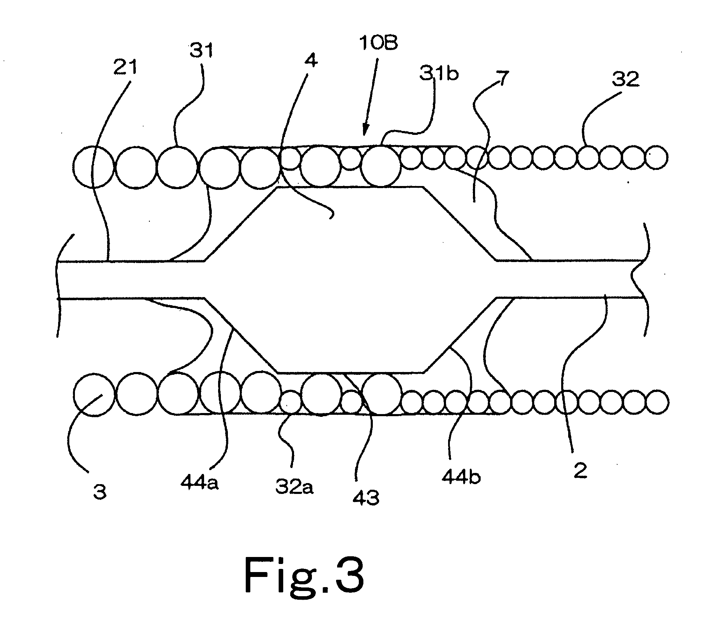

Fig. 3 is a partial enlarged sectional view of another embodiment of the guide wire according to the present invention. Fig. 3 is an enlarged sectional view of a projection 4 and the vicinity thereof. This guide wire 10B is the same as the above-described guide wire 1 in basic configuration; therefore, the differences therebetween will be described principally, and, for the other points, the above description can be referred to.

In the embodiment shown in Fig. 3, a projecting portion of the projection 4 projecting concentrically relative to the axis is trapezoidal in sectional shape. As shown in Fig. 3, the projection 4 has a cylindrical portion 43. The cylindrical portion 43 is parallel to the axis of a core member 2. In addition, the projection 4 has tapered portions 44a and 44b respectively on the distal side and the proximal side thereof. A proximal end portion 31b of a distal side coil 31 and a distal end portion 32a of a proximal side coil 32 are entangled with each other. The projection 4 is located at the entangled portion of the distal side coil 31 and the proximal side coil 32. The outside diameter of the cylindrical portion 43 of the projection 4 is substantially equal to the inside diameter of the distal side coil 31. In addition, the outside diameter of the cylindrical portion 43 is smaller than the inside diameter of the proximal side coil 32. The distal side coil 31 and the proximal side coil 32 are substantially the same in outside diameter. Since a difference in inside diameter is present between both the coils, a spiral space is formed on the outside surface of the cylindrical portion 43, which promises easy flow-around of a brazing filler metal 7.

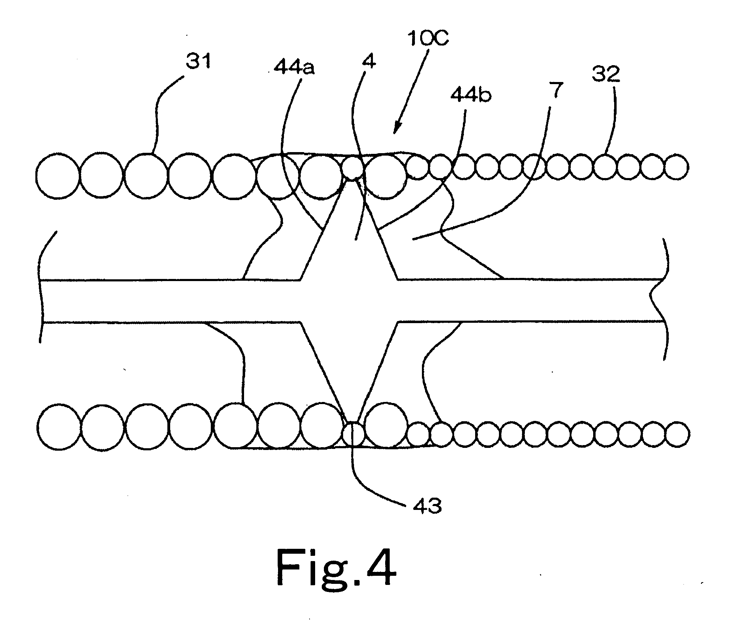

Fig. 4 is a partial enlarged sectional view of another embodiment of the guide wire according to the present invention. Fig. 4 is an enlarged sectional view of a projection 4 and the vicinity thereof. This guide wire 10C is the same as the above guide wires in basic configuration; therefore, the differences therebetween will be described principally, and, for the other points, the above description can be referred to.

In the embodiment shown in Fig. 4, a projecting portion of the projection 4 projecting concentrically relative to the axis is mount-like in sectional shape. The vertical section of the projection 4 is like a bead on a Japanese abacus in shape. The projection 4 has a cylindrical portion 43. The projection 4 has tapered portions 44a and 44b respectively on the distal side and the proximal side thereof. The whole length in the axial direction of the projection 4 is preferably smaller than the outside diameter of a larger-diameter core member 2 on the distal side or the proximal side of the projection 4. A proximal end portion 31b of a distal side coil 31 and a distal end portion 32a of a proximal side coil 32 are entangled with each other. The projection 4 is located at the entangled portion of the distal side coil 31 and the proximal side coil 32. The outside diameter of the cylindrical portion 43 of the projection 4 is substantially equal to the inside diameter of the proximal side coil 32. In addition, the outside diameter of the cylindrical portion 43 is greater than the inside diameter of the distal side coil 31. The distal side coil 31 and the proximal side coil 32 are substantially the same in outside diameter. The projection 4 may be free of the cylindrical portion 43, and may be like a hanging bell in sectional shape, with its outside diameter varying gradually. The length in the axial direction of the cylindrical portion 43 of the projection 4 is smaller than the diameter of the elementary wire of the proximal side coil 32. However, the length in the axial direction of the cylindrical portion 43 may be substantially equal to the diameter of the elementary wire of the proximal side coil 32. In addition, the length in the axial direction of the cylindrical portion 43 may be greater than the diameter of the elementary wire of the proximal side coil 32 and smaller than the diameter of the elementary wire of the distal side coil 31.

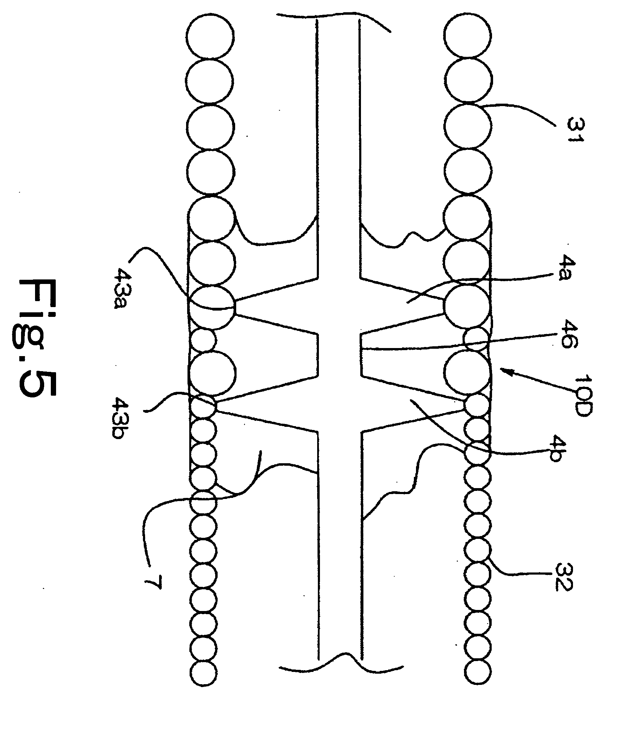

Fig. 5 is a partial enlarged sectional view of another embodiment of the guide wire according to the present invention. Fig. 5 is an enlarged sectional view of projections 4 and the vicinity thereof. This guide wire 10D is the same as the above-described guide wires in basic configuration; therefore, the differences therebetween will be described principally, and, for the other points, the above description can be referred to.

In the embodiment shown in Fig. 5, as the projections projecting concentrically relative to the axis, two projections 4a and 4b are provided. Projecting portions of the projection 4a and the projection 4b are mount-like in sectional shape. The sections of the projection 4a and the projection 4b are like a bead on a Japanese abacus in shape. The projection 4a and the projection 4b have a cylindrical portion 43a and a cylindrical portion 43b, respectively. As shown in the figure, the projecting portions of the projection 4a and the projection 4b are substantially the same in sectional shape, but the cylindrical portion 43a of the projection 4a on the distal side may be longer than the cylindrical portion 43b of the projection 4b on the proximal side. Alternatively, the cylindrical portion 43b may be longer than the cylindrical portion 43a. The diameter of the elementary wire of a distal side coil 31 is greater than the diameter of the elementary wire of a proximal side coil 31. The cylindrical portion 43a of the distal side projection 4a is desirably substantially equal to or greater than the elementary wire diameter of the distal side coil 31. The cylindrical portion 43b of the proximal side projection 4b is desirably substantially equal to or greater than the elementary wire diameter of the proximal side coil 32. A proximal end portion 31b of the distal side coil 31 and a distal end portion 32a of the proximal side coil 32 are entangled with each other. The proximal end portion 31b of the distal side coil 31 and the distal end portion 32a of the proximal side coil 32 may not necessarily be entangled with each other, and the proximal end portion 31b and the distal end portion 32a may be fixed in the state of being adjacent to each other. The projection 4a and the projection 4b are located at the entangled portion of the distal side coil 31 and the proximal side coil 32. The outside diameter of the cylindrical portion 43a is substantially equal to the inside diameter of the distal side coil 31. In addition, the outside diameter of the cylindrical portion 43b is substantially equal to the inside diameter of the proximal side coil 32. The distal side coil 31 and the proximal side coil 32 are substantially the same in outside diameter. The distance between the projection 4a and the projection 4b is equivalent to a value in the range of one turn to three turns of the distal side coil 31. A valley portion 46 between the projection 4a and the projection 4b has an outside diameter substantially equal to that of a distal end portion 21 of a core member 2. The outside diameter of the valley portion 46 may be greater than the outside diameter of the distal end portion 21 of the core member 2. In addition, the outside diameter of the valley portion 46 may be smaller than the outside diameter of the distal end portion 21 of the core member 2, for securing flexibility. The valley portion 46 is filled with a brazing filler metal 7, which is an adhesion material. The brazing filler metal 7 may be provided to fill the valley portion 46 in such a manner as to leave a space between the distal side coil 31 and the proximal side coil 32.

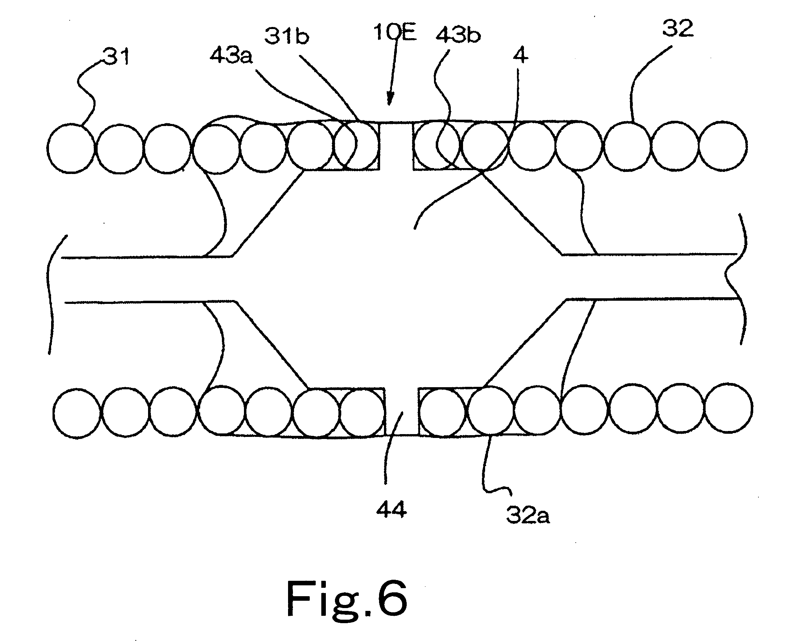

Fig. 6 is a partial enlarged sectional view of another embodiment of the guide wire according to the present invention. Fig. 6 is an enlarged sectional view of a projection 4 and the vicinity thereof. This guide wire 10E is the same as the above-described guide wires in basic configuration; therefore, the differences therebetween will be described principally, and, for the other points, the above description can be referred to.

In the embodiment shown in Fig. 6, a projecting portion of the projection 4 projecting concentrically relative to the axis is trapezoidal in sectional shape. The projection 4 is provided a projected portion 44 at substantially the center of the outside surface thereof. A cylindrical portion 43a and a cylindrical portion 43b are provided respectively on the distal side and the proximal side of the projected portion 44. The outside diameters of the cylindrical portion 43a and the cylindrical portion 43b of the projection 4 are substantially equal to the inside diameters of a distal side coil 31 and a proximal side coil 32. The outside diameters of the distal side coil 31 and the proximal side coil 32 are substantially equal. A proximal end portion 31b of the distal side coil 31 is terminated on the distal side portion of the projected portion 44. A distal end portion 32a of the proximal side coil 32 is terminated on the proximal side portion of the projected portion. With the projected portion 44 provided, the distal side coil 31 and the proximal side coil 32 can be disposed concentrically with a core member 2 without entangling them with each other, and the proximal end portion 31b of the distal side coil 31 and the distal end portion 32a of the proximal side coil 32 can be located accurately and easily. The outside diameter of the projected portion 44 is substantially equal to the outside diameters of the distal side coil 31 and the proximal side coil 32. While the sectional shape of the projected portion 44 is rectangular in Fig. 6, the sectional shape may be a shape with an outside diameter varying gradually, for example, a mount-like shape or a hanging bell-like shape. The projected portion 44 is formed integrally with the projection 4 and of the same material. The projected portion 44 may be a separate member. For example, the projected portion 44 may be formed by a method in which an annular body having an inside diameter substantially equal to the outside diameter of the cylindrical portion 43a or 43b is mounted on the cylindrical portion, and is fixed to the latter by an appropriate joining method.

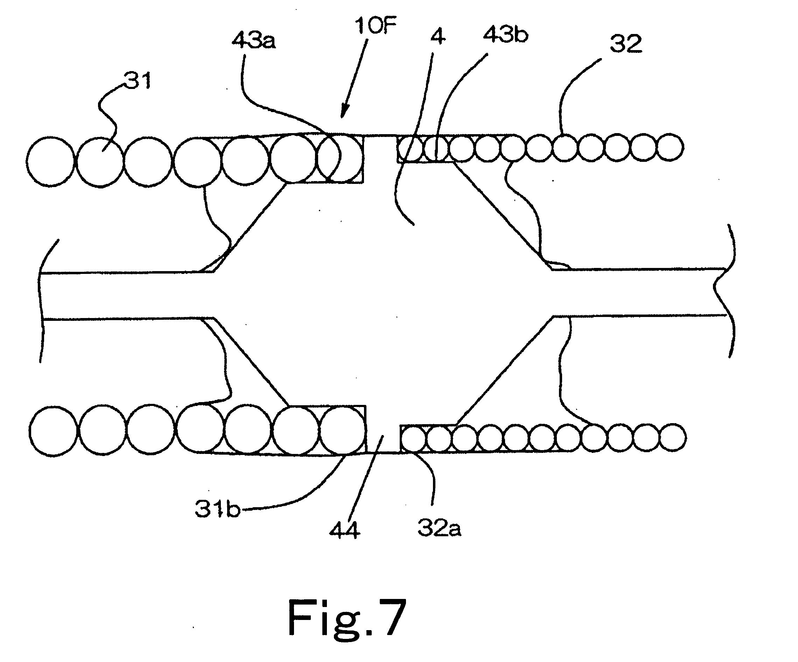

Fig. 7 is a partial enlarged sectional view of another embodiment of the guide wire according to the present invention. Fig. 7 is an enlarged sectional view of a projection 4 and the vicinity thereof. This guide wire 10F is the same as the above-described guide wires in basic configuration; therefore, the differences therebetween will be described principally, and, for the other points, the above description can be referred to.

In the embodiment shown in Fig. 7, a projecting portion of the projection 4 projecting concentrically relative to the axis is trapezoidal in sectional shape. The projection 4 is provided with a projected portion 44 at substantially the center of the outside surface thereof. A cylindrical portion 43a and a cylindrical portion 43b are provided respectively at the distal side and the proximal side of the projected portion 44. The outside diameter of the cylindrical portion 43a of the projection 4 is substantially equal to the inside diameter of a distal side coil 31. The outside diameter of the cylindrical portion 43b is substantially equal to the inside diameter of a proximal side coil 32. The outside diameters of the distal side coil 31 and the proximal side coil 32 are substantially equal. A proximal end portion 31b of the distal side coil 31 is terminated on the distal side portion of the projected portion 44. A distal end portion 32a of the proximal side coil 32 is terminated on the proximal side portion of the projected portion 44. With the projected portion 44 provided, the distal side coil 31 and the proximal side coil 32 can be disposed concentrically with the core member 2 without entangling them with each other, and the proximal end portion 31b of the distal side coil 31 and the distal end portion 32a of the proximal side coil 32 can be located accurately. The outside diameter of the projected portion 44 is substantially equal to the outside diameters of the distal side coil 31 and the proximal side coil 32. While the sectional shape of the projected portion 44 is rectangular in Fig. 7, the shape may be a shape with an outside diameter varying gradually, for example, a mount-like shape or a hanging bell-like shape. While the projected portion 44 is provided at substantially the center of the outside surface of the projection 4, the projected portion may be provided at a position deviated to the distal side or the proximal side from the substantially central position. A length of the distal cylindrical portion 43a may be shorter than a length of the proximal cylindrical portion 43b. The length of the proximal cylindrical portion 43b may be shorter than the length of the distal cylindrical portion 43a.

The projected portion 44 is formed integrally with the projection 4 and of the same material. The projected portion 44 may be a separate member. For example, the projected portion 44 may be formed by a method in which an annular body having an inside diameter substantially equal to the outside diameter of the cylindrical portion 43a or the cylindrical portion 43b is mounted on the cylindrical portion 43a or the cylindrical portion 43b, and is fixed to the cylindrical portion by an appropriate joining method.

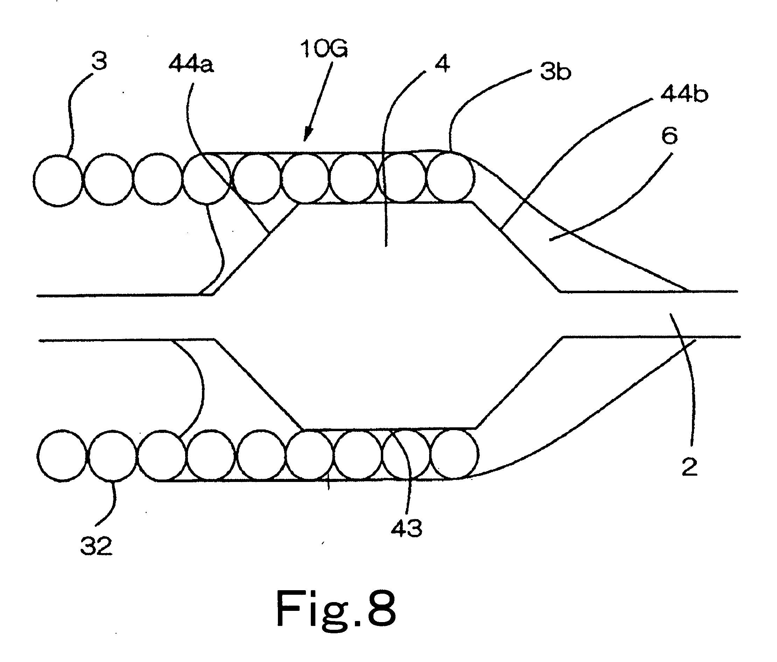

Fig. 8 is a partial enlarged sectional view of another embodiment of the guide wire according to the present invention. Fig. 8 is an enlarged sectional view of a projection 4 and the vicinity thereof. This guide wire 10G is the same as the above-described guide wires in basic configuration; therefore, the differences therebetween will be described principally, and, for the other points, the above description can be referred to.

In the embodiment shown in Fig. 8, the projection 4 projecting concentrically relative to the axis is provided at a proximal end portion 3b of a coil 3. The projection 4 is provided at a constant outside diameter portion of a core member 2. The projection 4 may be provided at a tapered portion. A projecting portion of the projection 4 is trapezoidal in sectional shape. The projection 4 has a cylindrical portion 43. In addition, the projection 4 has tapered portions 44a and 44b on the distal side and the proximal side thereof. The outside diameter of the cylindrical portion 43 of the projection 4 is substantially equal to the inside diameter of the coil 3. An adhesion material 6 is provided to fill the gaps between loops of the elementary wire at the proximal end portion 3b of the coil 3 and the gap between the cylindrical portion 43 of the projection 4 and the coil 3, to thereby fix the proximal end portion 3b of the coil 3 and the core member 2. The adhesion material 6 covers the tapered portions 44a and 44b of the projection 4. The outside diameter of the core member 2 on the proximal side of the projection 4 is equal to the outside diameter of the core member 2 on the distal side of the projection 4, but the outside diameter of the core member 2 on the proximal side of the projection 4 may be greater than the outside diameter of the core member 2 on the distal side of the projection 4. The length in the axial direction of the cylindrical portion 43 of the projection 4 is greater than the lengths in the axial direction of the tapered portions 44a and 44b. The length in the axial length of at least one of the tapered portion 44a and the tapered portion 44b may be greater than the length in the axial direction of the cylindrical portion 43. A length of the distal tapered portion 44a may be shorter than a length of the proximal tapered portion 44b. The length of the proximal tapered portion 44b may be shorter than the length of the distal tapered portion 44a.

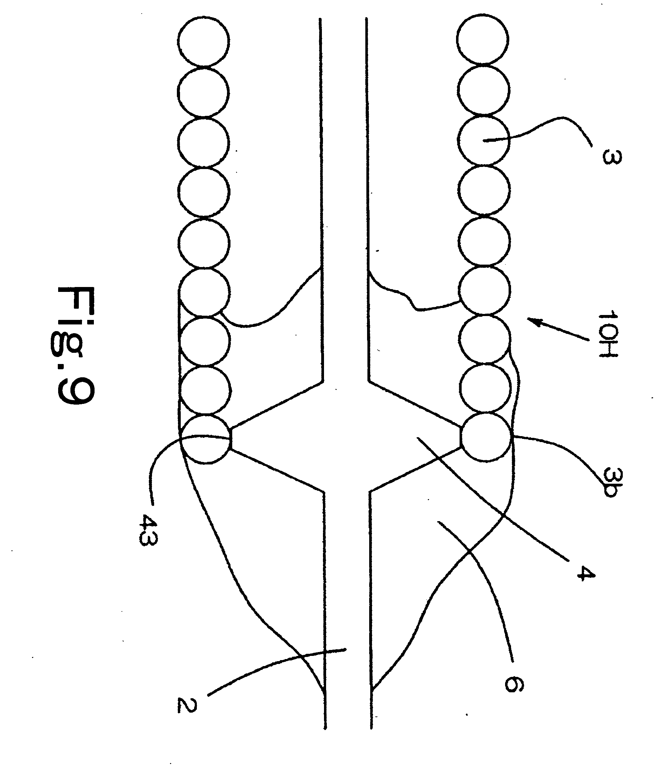

Fig. 9 is a partial enlarged sectional view of another embodiment of the guide wire according to the present invention. Fig. 9 is an enlarged sectional view of a projection 4 and the vicinity thereof. This guide wire 10H is the same as the above-described guide wires in basic configuration; therefore, the differences therebetween will be described principally, and, for the other points, the above description can be referred to.

In the embodiment shown in Fig. 9, the projection 4 projecting concentrically relative to the axis is provided at a proximal end portion 3b of a coil 3. A projecting portion of the projection 4 is mount-like in sectional shape. The section of the projection 4 is like a bead on a Japanese abacus in shape. The projection 4 has a cylindrical portion 43. The outside diameter of the cylindrical portion 43 of the projection 4 is substantially equal to the inside diameter of the proximal end portion 3b of the coil 3. The length in the axial direction of the cylindrical portion 43 of the projection 4 is smaller than the diameter of the elementary wire of the coil 3. However, the length in the axial direction of the cylindrical portion 43 may be substantially equal to the diameter of the elementary wire of the coil 3. In addition, the length in the axial direction of the cylindrical portion 43 is preferably smaller than the outside diameter of the core member 2.

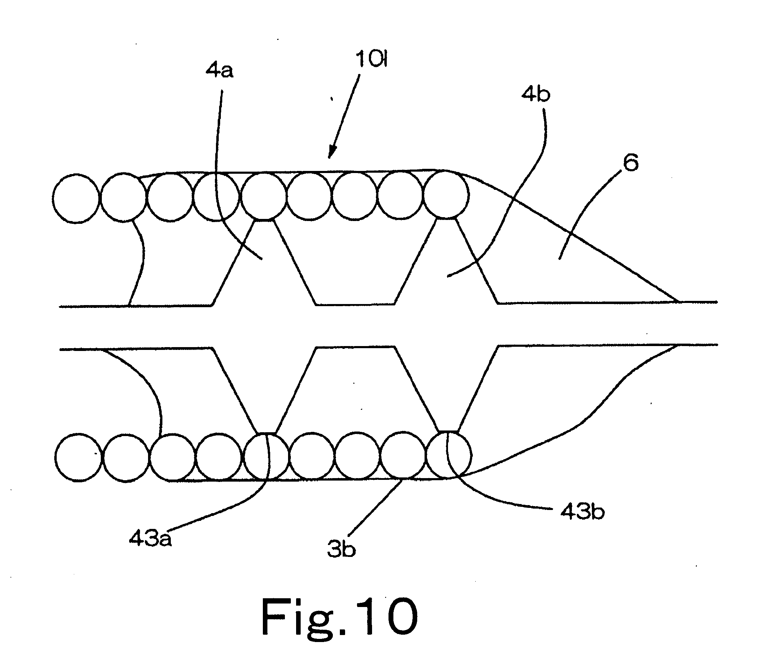

Fig. 10 is a partial enlarged sectional view of another embodiment of the guide wire according to the present invention. Fig. 10 is an enlarged sectional view of projections 4 and the vicinity thereof. This guide wire 10I is the same as the above-described guide wires in basic configuration; therefore, the differences therebetween will be described principally, and, for the other points, the above description can be referred to.

In the embodiment shown in Fig. 10, the projections 4 projecting concentrically relative to the axis are provided at a proximal end portion 3b of a coil 3. As the projection projecting concentrically relative to the axis, two projections 4a and 4b are provided. Projecting portions of the projection 4a and the projection 4b are mount-like in sectional shape. The sections of the projection 4a and the projection 4b are like a bead on a Japanese abacus in shape. The projection 4a and the projection 4b have a cylindrical portion 43a and a cylindrical portion 43b, respectively. While the sectional shapes of the projecting portions of the projection 4a and the projection 4b are substantially the same, as shown in the figure, the cylindrical portion 43a of the projection 4a on the distal side may be longer than the cylindrical portion 43b of the projection 4b on the proximal side. In addition, the cylindrical portion 43b may be longer than the cylindrical portion 43a. The outside diameters of the cylindrical portions 43 of the projections 4 are substantially equal to the inside diameter of the proximal end portion 3b of the coil 3. The distance between the projection 4a and the projection 4b is equivalent to a value in the range of one turn to three turns of the distal side coil 31.

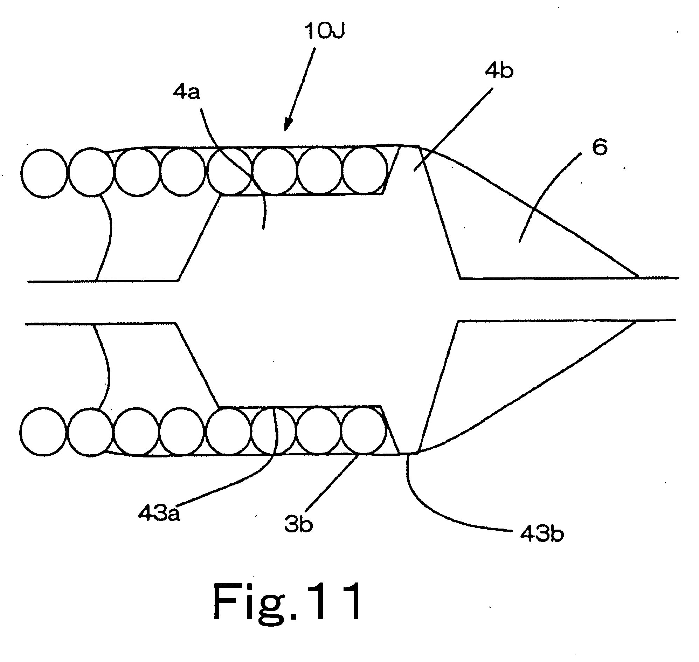

Fig. 11 is a partial enlarged sectional view of another embodiment of the guide wire according to the present invention. Fig. 11 is an enlarged sectional view of a projection 4 and the vicinity thereof. This guide wire 10J is the same as the above-described guide wires in basic configuration; therefore, the differences therebetween will be described principally, and, for the other points, the above description can be referred to.

In the embodiment shown in Fig. 11, the projection 4 projecting concentrically relative to the axis is provided at a proximal end portion 3b of a coil 3. As the projection protecting concentrically relative to the axis, a projection 4a is provided. Furthermore, the projection 4a is provided with a protected portion 4b at a proximal end portion thereof. The projection 4a and the projected portion 4b have a cylindrical portion 43a and a cylindrical portion 43b, respectively. The outside diameter of the cylindrical portion 43a of the projection 4 is substantially equal to the inside diameter of the proximal end portion 3b of the coil 3. The outside diameter of the projected portion 4b is greater than the inside diameter of the proximal end portion 3b of the coil 3. This configuration permits the coil 3 to be easily positioned in the axial direction. The outside diameter of the projected portion 4b is preferably substantially equal to or smaller than the outside diameter of the proximal end portion 3b of the coil 3. While the projected portion 4b is trapezoidal in section, it may lack the cylindrical portion 43b; for example, the sectional shape may be like a hanging bell or the above described.

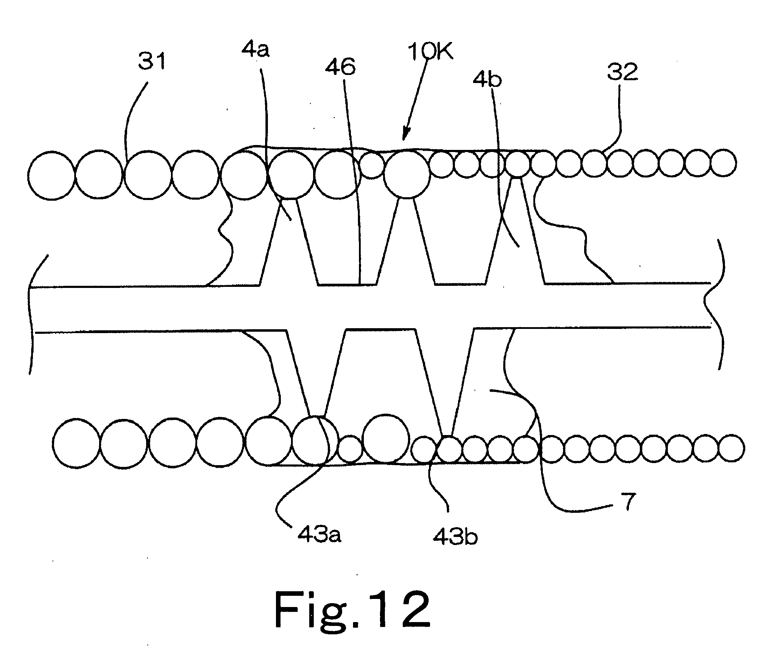

Fig. 12 is a partial enlarged sectional view of another embodiment of the guide wire according to the present invention. Fig. 12 is an enlarged sectional view of a projection 4 and the vicinity thereof. This guide wire 10K is the same as the above-described guide wires in basic configuration; therefore, the differences therebetween will be described principally, and, for the other point, the above description can be referred to.

In the embodiment shown in Fig. 12, a projecting portion of the projection 4 projecting concentrically relative to the axis is trapezoidal in sectional shape and the projection is provided spirally. As shown in Fig. 12, the projection 4 has a cylindrical portion 43. The cylindrical portion 43 is parallel to the axis of a core member 2. The inside diameter of a distal side coil 31 is substantially equal to the outside diameter of a cylindrical portion 43a of a distal side projection 4a, and the inside diameter of a proximal side coil 32 is substantially equal to the outside diameter of a cylindrical portion 43b of a proximal side projection 4b. With the projection provided spirally, a spiral space is formed, which promises easy flow-around of a brazing filler metal 7. The spiral projection 4a is accompanied by a spiral valley portion 46. The valley portion 46 has an outside diameter substantially equal to that of a distal end portion 21 of the core member 2. The outside diameter of the valley portion 46 may be greater than the outside diameter of the distal end portion 21 of the core member 2. In addition, the outside diameter of the valley portion 46 may be smaller than the outside diameter of the distal end portion 21 of the core member 2, for securing flexibility. The valley portion 46 is filled with the brazing filler metal 7, which is an adhesion material. The brazing filler metal 7 may be provided to fill the valley 46 in such a manner as to leave a space between itself and the distal side coil 31 and the proximal side coil 32.

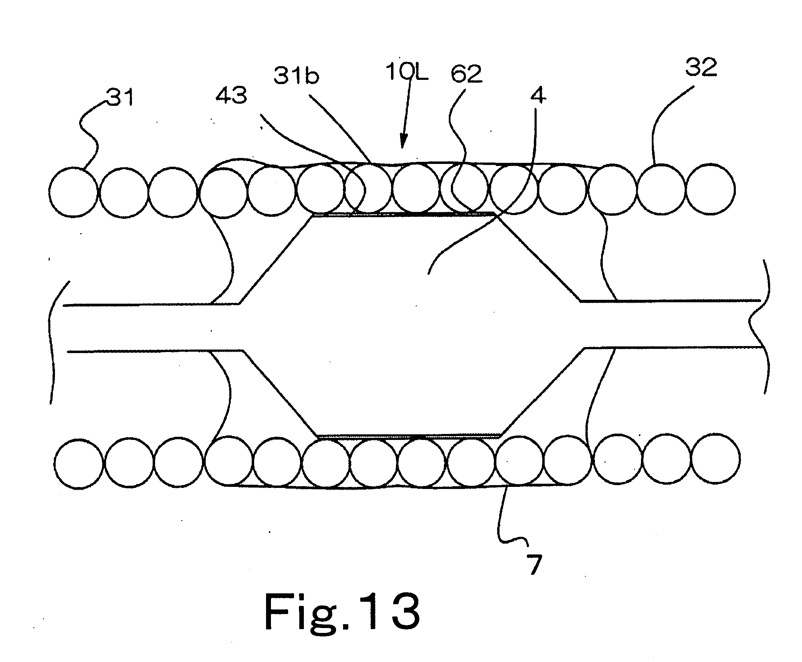

Fig. 13 is a partial enlarged sectional view of another embodiment of the guide wire according to the present invention. Fig. 13 is an enlarged sectional view of a projection 4 and the vicinity thereof. This guide wire 10L is the same as the above-described guide wires in basic configuration; therefore, the differences therebetween will be described principally, and, for the other points, the above description can be referred to.

In the embodiment shown in Fig. 13, a projecting portion of the projection 4 projecting concentrically relative to the axis is trapezoidal in sectional shape. As shown in Fig. 13, the projection 4 has a cylindrical portion 43. The cylindrical portion 43 is parallel to the axis of a core member 2. The guide wire 10L is provided with a metal layer 62 on the surface of the cylindrical portion 43 of the projection 4. As the material constituting the metal layer 62, a material can be selected such that the adhesion between the metal layer 62 and a brazing filler metal 7 is higher than the adhesion between the core metal 2 and the brazing filler metal 7. The metal layer 62 can be formed by plating or vapor deposition. A coil 3 may be fixed to the projection 4 by welding. Laser welding and TIG welding can be used for the welding. A metallic annular member may be interposed between the coil 3 and the projection 4. A part of the coil 3 and the surface of the metallic annular member are united integrally through melting and solidification, whereby the coil 3 is fixed to the projection 4.

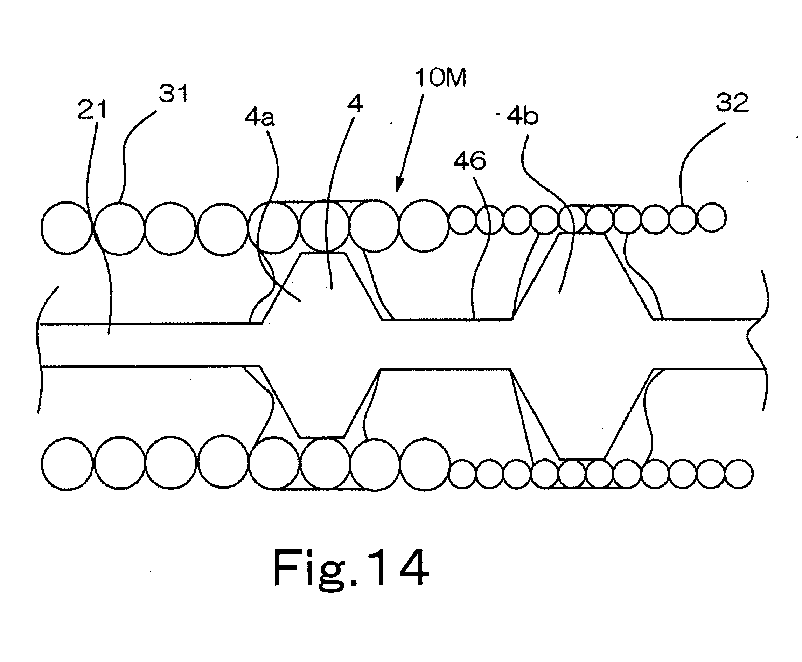

Fig. 14 is a partial enlarged sectional view of another embodiment of the guide wire according to the present invention. Fig. 14 is an enlarged sectional view of projections 4 and the vicinity thereof. This guide wire 10M is the same as the above-described guide wires in basic configuration; therefore, the differences therebetween will be described principally, and, for the other points, the above description can be referred to.

In the embodiment shown in Fig. 14, as projections projecting concentrically relative to the axis, two projections, i.e., a distal side projection 4a and a proximal side projection 4b are provided. Projecting portions of the projection 4a and the projection 4b are mount-like in sectional shape. As shown in the figure, the sectional shapes of the projecting portions of the projection 4a and the projection 4b are substantially the same. A proximal end portion 31b of a distal side coil 31 and a distal end portion 32a of a proximal side coil 32 are joined to each other. The joint portion (not shown) of the distal side coil 31 and the proximal side coil 32 is located between the projection 4a and the projection 4b. A valley portion 46 between the projection 4a and the projection 4b has an outside diameter substantially equal to a distal end portion 21 of a core member 2. The outside diameter of the valley portion 46 may be greater than the outside diameter of the distal end portion 21 of the core member 2. In addition, the outside diameter of the valley portion 46 may be smaller than the outside diameter of the distal end portion 21 of the core member 2, for securing flexibility. The valley portion 46 is substantially not filled with a brazing filler metal 7, which is an adhesion material. A space is formed between the distal side coil 31, the proximal side coil 32 and the valley portion 46.

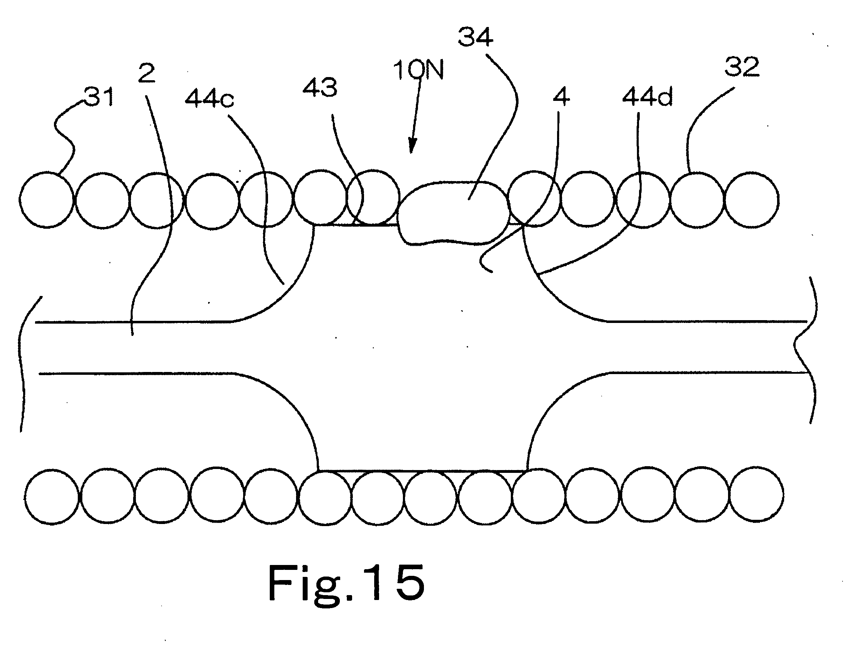

Fig. 15 is a partial enlarged sectional view of another embodiment of the guide wire according to the present invention. Fig. 15 is an enlarged sectional view of a projection 4 and the vicinity thereof. This guide wire 10N is the same as the above-described guide wires in basic configuration; therefore, the differences therebetween will be described principally, and, for the other points, the above description can be referred to.

In the embodiment shown in Fig. 15, a projecting portion of the projection 4 projecting concentrically relative to the axis is trapezoidal in sectional shape. As shown in Fig. 15, the projection 4 has a cylindrical portion 43. The cylindrical portion 43 is parallel to the axis of a core member 2. The cylindrical portion 43 is provided with concave portions 44c and 44d on the distal side and the proximal side. The cylindrical portion 43 may be provided with the concave portion 44c only on the distal side. Alternatively, the cylindrical portion 43 may be provided with the concave portion 44d only on the proximal side. The distal side of the concave portion 44c and the proximal side of the concave portion 44d are gradually continued to the surface of the core member 2. A distal side coil 31 and a proximal side coil 32 are fixed to the projection 4 at a fixation portion 34. The fixation portion 34 is preferably a mass of the mixture of the materials of the distal side coil 31, the proximal side coil 32 and the projection 4. The fixation portion 34 can be formed by irradiating the boundary between the distal side coil 31 and the proximal side coil 32 with laser light so as to melt the materials of the distal side coil 31, the proximal side coil 32 and the projection 4. The distal side coil 31 and the proximal side coil 32 are fixed to the projection 4 by welding. A plurality of the fixation portions 34 may be provided.

The fixation portions 34 can be formed on each the distal side coil 31 and the proximal side coil 32. Where a plurality of the fixation portions 34 are provided, they are preferably provided symmetrically or at regular intervals with respect to the circumferential direction around the axis of the core member 2. While the coils 31 and 32 as separate members have been described in relation to the guide wire 10N, a single-member coil may be fixed to the projection 4 at the fixation portion(s) 34. The fixation portion(s) 34 may be constituted of a brazing filler metal filling the gaps between the distal side coil 31, the proximal side coil 32 and the projection 4.

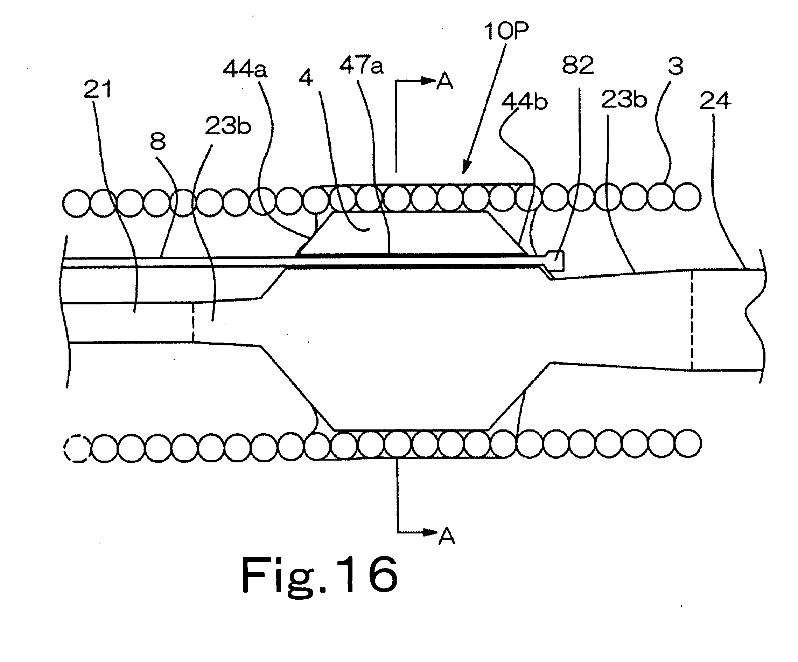

Fig. 16 is a partial enlarged sectional view of another embodiment of the guide wire according to the present invention. Fig. 16 is an enlarged sectional view of a projection 4 and the vicinity thereof. This guide wire 10P is the same as the above-described guide wires in basic configuration; therefore, the differences therebetween will be described principally, and, for the other points, the above description can be referred to.

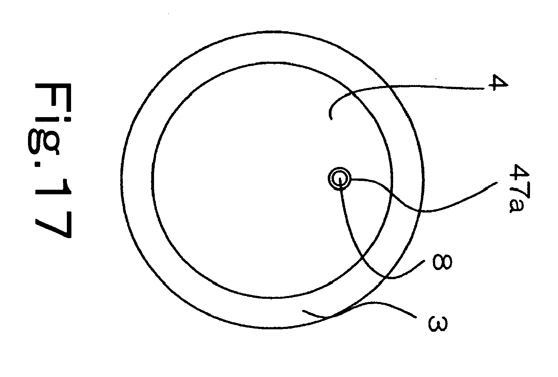

In the embodiment shown in Fig. 16, a projecting portion of the projection 4 projecting concentrically relative to the axis is trapezoidal in sectional shape. The guide wire 10P is provided in the projection 4 with a hole 47a extending in the axial direction. As shown in Fig. 16, the hole 47a is preferably penetrating through the projection 4. A shaping member 8 is secured to the projection 4. The hole 47a is preferably provided in parallel with the axial direction. As shown in Fig. 17, which is a sectional view along line A-A of Fig. 16, the shaping member 8 is passed through the hole 47a. The shaping member 8 has the function of reshaping a distal end portion of the guide wire. A distal end portion of the shaping member 8 extends to the most distal end (not shown) of the guide wire, and is fixed to the most distal end of the coil 3 by a brazing filler metal. A proximal end portion of the shaping member 8 has a lock portion 82, which prevents the shaping member 8 from slipping off from the projection 4. The lock portion 82 has an outside diameter greater than the inside diameter of the hole 47a. Another embodiment of the lock portion 82 is a flat plate portion having a width greater than the inside diameter of the hole 47a.

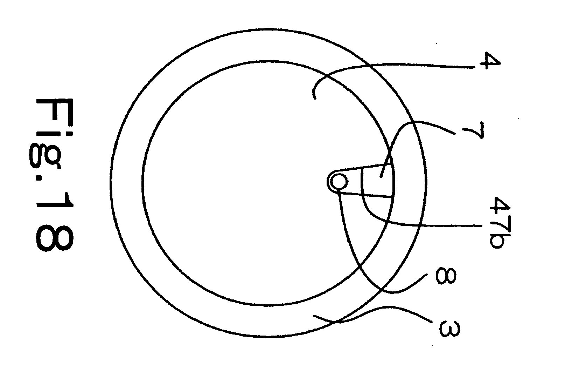

While the projection 4 is provided with the hole 47a extending in the axial direction in Fig.. 17, a modified example of the hole 47a is shown in Fig. 18, in which the projection 4 is provided with a groove 47b extending in the axial direction. While the groove 47b is provided over the whole length of the projection 4, the groove 47b may be provided in a distal side part of the projection 4. The groove 47b is preferably provided in parallel to the axial direction. A proximal end portion of the shaping member 8 is located at the groove 47b. The groove 47b is filled with a brazing filler metal 7 for fixing the shaping member 8.

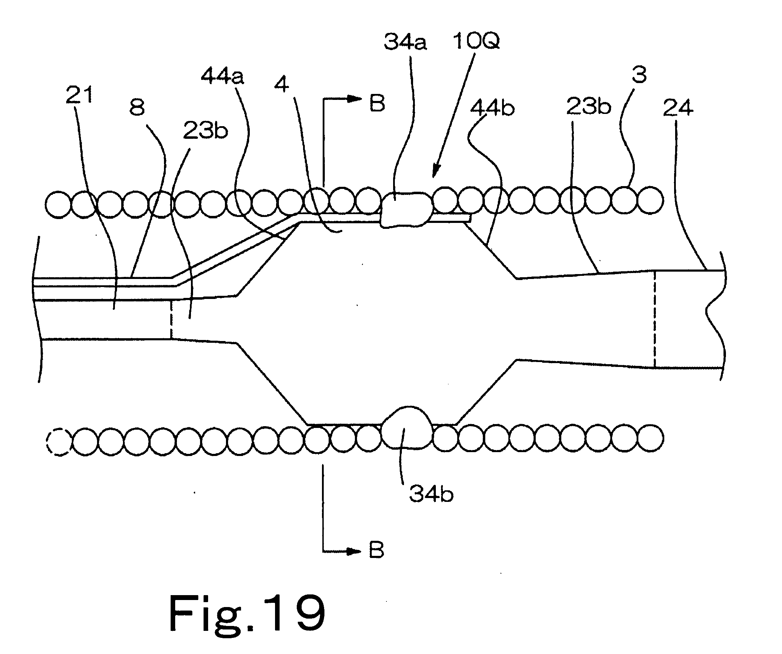

Fig. 19 is a partial enlarged sectional view of another embodiment of the guide wire according to the present invention. Fig. 19 is an enlarged sectional view of a projection 4 and the vicinity thereof. This guide wire 10Q is the same as the above-described guide wires in basic configuration; therefore, the differences therebetween will be described principally, and, for the other points, the above description can be referred to.

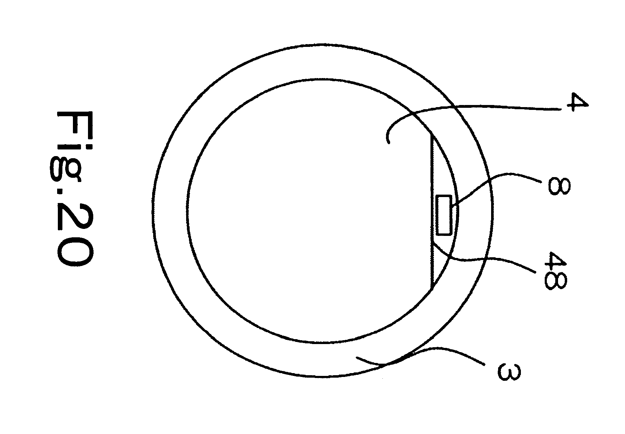

In the embodiment as shown in Fig. 19, a projecting portion of the projection 4 projecting concentrically relative to the axis is trapezoidal in sectional shape. The guide wire 10Q is provided with a shaping member 8 between the projection 4 and a coil 3. The shaping member 8 has the function of reshaping a distal end portion of the guide wire. The shaping member 8 is secured to the projection 4. The shaping member 8 is flat plate-like in sectional shape. A distal end portion (not shown) of the shaping member 8 extends to the most distal end of the guide wire, and is fixed to the most distal end of the coil 3 by a brazing filler metal. As shown in Fig. 20, which is a sectional view along line B-B of Fig. 19, the projection 4 is provided with a shaping member mount portion 48, on which the shaping member 8 is mounted. A proximal end portion of the shaping member 8 is provided in parallel with a hollow cylindrical portion of the projection 4. The shaping member mount portion 48 has a flat surface provided in the axial direction at the hollow cylindrical portion of the projection 4. In addition, the flat surface may be replaced by a recessed portion provided in the axial direction in the hollow cylindrical portion of the projection 4 of the shaping member 8. The shaping member 8 and the coil 3 are fixed to the projection 4 at a fixation portion 34a. The fixation portion 34a is preferably a mass of the mixture of at least the materials of the coil 3, the shaping member 8. The fixation portion 34a may be a mass of the mixture of the materials of the coil 3, the shaping member 8 and the projection 4. The fixation portion 34a can be formed by irradiating the coil 3 with laser light so as to melt the coil 3, the shaping member 8 and the projection 4. The fixation portion 34a may be formed by irradiating the coil 3 with laser light so as to melt the coil 3, the shaping member 8.

The coil 3 and the shaping member 8 are fixed to the projection 4 by welding. Other than the fixation portion 34a, a fixation portion 34b is provided, where the coil 3 and the projection 4 are fixed to each other. The fixation portion 34b is preferably a mass of the mixture of the materials of the coil 3 and the projection 4. The fixation portion 34b can be formed by irradiating the coil 3 with laser light so as to melt the coil 3 and the projection 4. A plurality of the fixation portions 34b may be provided. Where pluralities of the fixation portions 34a and the fixation portions 34b are provided, they are preferably provided symmetrically or at regular intervals with respect to the circumferential direction around the axis of the core member 2.

The above-described embodiments may be combined in various manners. For example, any of the above-described guide wires 1, 10A, 10B, 10C, 10D, 10E, 10F, 10K, 10L, 10M, 10N, 10P and 10Q showing the embodiments wherein the coil is fixed to the core member at a part between the distal end portion and the proximal end portion of the coil can be combined with any of the above-described guide wires 10G, 10H, 10I and 10J showing the embodiments wherein the coil is fixed to the core member at the proximal end portion of the coil.

The above projections are formed concentrically circle in cross section, but projection in the above-described embodiments may be oval, triangle, square, rectangular, pentagon, hexagon, or polygon in cross section.

The present invention is not limited to the details of the above described embodiments. The scope of the invention is defined by the appended claims and all changes and modifications as fall within the equivalence of the scope of the claims are therefore to be embraced by the invention.

## Claims

1. A guide wire comprising a core member having at least one projection projecting concentrically relative to an axis, and a coil having a distal end portion and a proximal end portion and covering at least a distal side portion of said core member, wherein said coil is fixed to said projection.

2. The guide wire as set forth in claim 1, wherein said projection has a cylindrical portion.

3. The guide wire as set forth in claim 1, wherein said projection has a concave portion at least on one of the distal side and the proximal side thereof.

4. The guide wire as set forth in claim 1, wherein said projection has tapered portions on the distal side and the proximal side thereof.

5. The guide wire as set forth in claim 1, wherein a projecting portion of said projection is trapezoidal in sectional shape.

6. The guide wire as set forth in claim 1, wherein a projecting portion of said projection is mount-like in sectional shape.

7. The guide wire as set forth in claim 1, wherein said projection has an outside diameter substantially equal to the inside diameter of said coil.

8. The guide wire as set forth in any of claims 1 to 7, wherein said projection is provided at a portion between said distal portion and said proximal portion of said coil and/or at said proximal portion of said coil.

9. The guide wire as set forth in claim 1, wherein the projection is provided at a portion between the distal end portion and the proximal end portion of the coil and/or at the distal end portion of the coil.

10. The guide wire as set forth in claim1, wherein the coil includes a distal side coil and a proximal side coil, and the projection fixes a proximal end portion of the distal side coil and a distal end portion of the proximal side coil to the core member.

11. The guide wire as set forth in claim 10, wherein the distal side coil and the proximal side coil have substantially equal outside diameters and difference inside diameters, and the projection has a small-diameter portion and a large-diameter portion corresponding to the difference in inside diameter between the coils.

12. The guide wire as set forth in claim 11, wherein the projection has a concave portion on at least one of the distal side and the proximal side thereof.

13. The guide wire as set forth in claim 1, wherein two or more projections are provided.

14. The guide wire as set forth in claim 13, wherein the projection has a distal side projection and a proximal side projection, a proximal end portion of the distal side coil and a distal end portion of the proximal side coil are joined to each other, and the joint portion of the distal side coil and the proximal side coil is located between the distal side projection and the proximal side projection.

15. The guide wire as set forth in claim 1, wherein a shaping member is secured to the projection.
